# DEMANDE DE BREVET EUROPEEN

(11) **EP 4 755 866 A1**
(43) Date de publication de la demande: **10.06.2026**
(21) Numéro de dépôt: 25221498.6
(22) Date de dépôt: 12.06.2019
(51) Int. Cl.: C07C 17/04, C07C 17/20, C07C 17/25, C07C 21/18, C07C 17/087, C07C 19/045, C07C 17/06, C07C 19/05, C07C 19/12

(54) **MONOMERE DE DIFLUORURE DE VINYLIDENE BIOSOURCE ET POLYMERES LE CONTENANT**

(30) Priorité: 15.06.2018 FR 1855278
(62) Demande divisionnaire de: 19744762.6
(71) Demandeur: ARKEMA France, 92800 Puteaux (FR)
(72) Inventeur: BONNET, Anthony, 92800 PUTEAUX (FR); DUBOIS, Jean-Luc, 92800 PUTEAUX (FR)
(74) Mandataire: Arkema Patent

(57) **Abrégé**

L'invention concerne le difluorure de vinylidène biosourcé. L'invention concerne également des procédés de préparation du difluorure de vinylidène biosourcé à partir de diverses matières premières renouvelables. L'invention concerne aussi les homopolymères de difluorure de vinylidène obtenus par polymérisation dudit monomère, ainsi que les copolymères obtenus par copolymérisation dudit monomère avec un ou plusieurs co-monomères compatibles. L'invention se rapporte enfin à l'utilisation desdits homopolymères ou copolymères dans des applications telles que le génie chimique ou l'électronique, notamment des appareils électroniques grand public.

## Description

### DOMAINE DE L'INVENTION

La présente invention a pour objet le difluorure de vinylidène biosourcé. L'invention concerne également des procédés de préparation du difluorure de vinylidène biosourcé à partir de diverses matières premières renouvelables. L'invention concerne aussi les homopolymères de difluorure de vinylidène obtenus par polymérisation dudit monomère, ainsi que les copolymères obtenus par copolymérisation dudit monomère avec un ou plusieurs co-monomères compatibles. L'invention se rapporte enfin à l'utilisation desdits homopolymères ou copolymères dans toutes les applications où l'aspect de ces polymères est essentiel et/ou leurs propriétés dépendent de leur degré de pureté ; il s'agit des applications dans l'automobile, la filtration des fluides, notamment de l'eau, l'off-shore, le médical, le transport d'eau potable, le marché du semi-conducteur, la câblerie, les batteries Li-ion, le photovoltaïque, les articles de sport et les textiles pour le sport. Des applications préférées sont le génie chimique et l'électronique, notamment des appareils électroniques grand public.

### ARRIERE-PLAN TECHNIQUE

Le difluorure de vinylidène (1,1-difluoroéthylène ou VDF) est un gaz incolore, inodore et non toxique. Cette oléfine fluorée a l'avantage de ne pas contenir d'atome de chlore ou de brome, par conséquent sa toxicité est moindre comparée au chlorotrifluoroéthylène (CTFE) et bromotrifluoroéthylène (BrTFE). De plus, il ne possède pas le caractère explosif du tétrafluoroéthylène (TFE) et il est bien moins coûteux que le TFE, l'hexafluoropropène (HFP), le CTFE ou le BrTFE.

Le VDF est bien connu pour son utilisation comme monomère dans la fabrication du poly(fluorure de vinylidène) (PVDF) et comme co-monomère dans la fabrication de divers types de polymères fluorés.

Le VDF conventionnel est obtenu par vapocraquage ou craquage catalytique des coupes pétrolières. Il est synthétisé de façon industrielle par déshydrochloration pyrolytique du 1-chloro-1,1-difluoroéthane selon la réaction :

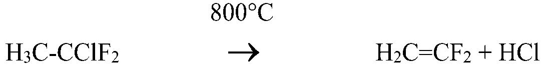

Le précurseur 1-chloro-1,1-difluoroéthane peut être préparé selon quatre voies:

H₃C-CCl₃ + 2 HF → H₃C-CClF₂ + 2 HCl

H₂C=CCl₂ + 2 HF → H₃C-CClF₂ + HCl

HC≡CH + 2 HF → H3C-CHF2

et

H3C-CHF2 + Cl₂ → H₃C-CClF₂ + HCl

CH₃-CHF₂ + Cl₂ → CH₃CClF₂ + HCl

D'autres voies de synthèse du VDF sont connues :
- la déshydrobromation du 1-bromo-1,1 difluoroéthane,
- la déshydrofluoration du 1,1,1-trifluoroéthane
- la déchloration du 1,2-dichloro-1,1-difluoroéthane
- la déshydrogénation/oxydéshydrogénation du 1,1-difluoroéthane

Le 1,1,2 trichloréthylène conduit par hydrofluoration à 1,2-dichloro-1,1-difluoroéthane, qui lui-même peut donner le difluoroéthylène par hydrogénolyse.

Les matières premières utilisées dans ces différents procédés de synthèse du VDF sont principalement d'origine fossile ou pétrolière. Ces procédés d'obtention contribuent ainsi à l'augmentation de l'effet de serre et sont considérés comme polluants pour l'environnement. Par ailleurs, les réserves mondiales de matières premières d'origine fossiles (pétrole et gaz naturel) ne cessent de diminuer, rendant impérative la recherche de nouvelles sources utilisables comme matières premières, qui soient renouvelables. Enfin, il a été constaté que les polymères fluorés fabriqués à partir du VDF conventionnel présentent des propriétés indésirables ; ils peuvent notamment contenir des impuretés qui augmentent l'indice de jaune (« yellowing index » ou YI) particulièrement redouté pour des applications comme le génie chimique ou encore les appareils électroniques grand public, où l'esthétique et le respect de la constante de la coloration sont exigés.

Il existe donc un besoin de disposer d'un difluorure de vinylidène préparé à partir de sources renouvelables et qui soit exempt de toute impureté pouvant affecter les propriétés des polymères préparés à partir de ce monomère, telles que les propriétés de vieillissement thermique ou l'indice de jaune.

Ce nouveau monomère biosourcé permet la fabrication des polymères fluorés qui combinent plusieurs avantages, à savoir ils associent les performances techniques et environnementales. La présente invention a donc pour but de concevoir de nouveaux polymères fluorés à base de VDF qui soient d'origine renouvelable et dont les performances soient au moins équivalentes à celles des polymères fluorés d'origine fossile.

### RESUME DE L'INVENTION

L'invention concerne en premier lieu le difluorure de vinylidène biosourcé. De manière caractéristique, la teneur en carbone renouvelable du VDF biosourcé est d'au moins 1% atomique, comme déterminé par la teneur en ¹⁴C selon la norme NF EN 16640 du 15 avril 2017. Ceci correspond à un ratio isotopique de ¹⁴C/¹²C dans la molécule de VDF d'au moins 1,2x10⁻¹⁴.

Selon un mode de réalisation, la teneur en carbone renouvelable dans le VDF biosourcé est supérieure à 5%, de préférence supérieure à 10%, de préférence supérieure à 25%, de préférence supérieure ou égale à 33%, de préférence supérieure à 50%, de préférence supérieure ou égale à 66%, de préférence supérieure à 75%, de préférence supérieure à 90%, de préférence supérieure à 95%, de préférence supérieure à 98%, de préférence supérieure à 99%, avantageusement égale à 100%.

L'invention a encore pour objet un procédé de préparation du composé selon l'invention, comprenant l'étape de fourniture d'éthylène biosourcé ayant une teneur en carbone renouvelable d'au moins 1%, et la transformation par synthèse en plusieurs étapes en difluorure de vinylidène biosourcé. La première étape de synthèse est la fabrication de chlorure de vinyle à partir de cet éthylène biosourcé. Plusieurs voies de synthèse sont ensuite possibles.

Selon un mode de réalisation, le chlorure de vinyle biosourcé est transformé en VDF biosourcé via les intermédiaires de synthèse suivants : 1,1,2-trichloroéthane, 1,1,1-trichloroéthane et 1-chloro-1,1-difluoroéthane.

Selon un mode de réalisation, le chlorure de vinyle biosourcé est transformé en VDF biosourcé via les intermédiaires de synthèse suivants : 1,1,2-trichloroéthane, 1,1-dichloroéthène, et 1-chloro-1,1-difluoroéthane.

Selon un mode de réalisation, le chlorure de vinyle biosourcé est transformé en VDF biosourcé via les intermédiaires de synthèse suivants : 1,1-dichloréthane, 1,1,1-trichloroéthane et 1-chloro-1,1-difluoroéthane.

Un autre objet de l'invention est un homopolymère de difluorure de vinylidène (PVDF) biosourcé préparé par polymérisation dudit monomère biosourcé.

L'invention concerne également des copolymères fluorés biosourcés obtenus par copolymérisation dudit monomère biosourcé avec un ou plusieurs co-monomères compatibles.

La présente invention a également pour objet l'utilisation du PVDF biosourcé et des copolymères fluorés biosourcés conformes à ceux définis précédemment dans diverses applications, telles que le génie chimique ou l'électronique, notamment des appareils électroniques grand public : équipement audio et vidéo pour usage domestique et commercial, jeux électroniques et équipement de divertissement, équipement de bowling et de billard, équipement de communication par câble et par satellite, composants électroniques utilisés dans les équipements audio et vidéo, équipement de télévision en circuit fermé et instruments de musique. D'une manière générale, les polymères fluorés biosourcés selon l'invention sont utilisés dans toutes les applications où l'aspect de ces polymères est essentiel et/ou leurs propriétés dépendent de leur degré de pureté ; il s'agit des applications dans l'automobile, la filtration des fluides, notamment de l'eau, l'off-shore, le médical, le transport d'eau potable, le marché du semi-conducteur, la câblerie, les batteries Li-ion, le photovoltaïque, les articles de sport et les textiles pour le sport.

Avantageusement, le PVDF biosourcé et/ou les copolymères fluorés biosourcés de l'invention sont utilisés seuls ou en mélange avec d'autres polymères, lesdits polymères fluorés biosourcés représentant en masse de 5 à 100%, de préférence de 5 à 70%, de préférence de 5 à 30%.

La présente invention permet de surmonter les inconvénients de l'état de la technique. Elle fournit plus particulièrement du difluorure de vinylidène biosourcé fabriqué à partir de matières premières biosourcées, répondant ainsi aux exigences du développement durable. Elle est particulièrement appropriée pour la fabrication de polymères fluorés pour des applications nécessitant des propriétés constantes et une haute pureté. Ainsi, la finalité des polymères fluorés biosourcés ne se limite pas à simplement remplacer les polymères fluorés d'origine fossile, mais de fournir des produits biosourcés de haute performance.

### DESCRIPTION DE MODES DE REALISATION DE L'INVENTION

L'invention est maintenant décrite plus en détail et de façon non limitative dans la description qui suit.

L'invention concerne selon un premier aspect le composé difluorure de vinylidène caractérisé en ce qu'il a un teneur en carbone renouvelable d'au moins 1% atomique.

Le carbone d'un biomatériau provient de la photosynthèse des plantes et donc du CO₂ atmosphérique. La dégradation (par dégradation, on entendra aussi la combustion/incinération en fin de vie) de ces matériaux en CO₂ ne contribue donc pas au réchauffement puisqu'il n'y a pas d'augmentation du carbone émis dans l'atmosphère. Le bilan CO₂ des biomatériaux est donc nettement meilleur et contribue à réduire l'empreinte carbone des produits obtenus (seule l'énergie pour la fabrication est à prendre en compte). Au contraire, un matériau d'origine fossile se dégradant aussi en CO₂ va contribuer à l'augmentation du taux de CO₂ et donc au réchauffement climatique.

Les composés selon l'invention auront donc une empreinte carbone qui sera meilleure que celle de composés obtenus à partir d'une source fossile.

L'invention améliore donc aussi le bilan écologique lors de la fabrication des monomères de difluorure de vinylidène et des polymères fluorés préparés à partir de ces monomères.

Le terme de « carbone renouvelable » indique que le carbone est d'origine naturelle et provient d'un biomatériau (ou de la biomasse), comme indiqué ci-après.

Le terme « biosourcé » signifie « issu de la biomasse ».

Un matériau d'origine renouvelable, appelé aussi biomatériau, est un matériau organique dans lequel le carbone provient de CO₂ fixé récemment (à l'échelle humaine) par photosynthèse à partir de l'atmosphère. Sur terre, ce CO₂ est capté ou fixé par les plantes. En mer, le CO₂ est capté ou fixé par des bactéries ou du plancton procédant à une photosynthèse. Un biomatériau (100% de carbone origine naturelle) présente un ratio isotopique ¹⁴C/¹²C supérieur à 10⁻¹², typiquement de l'ordre de 1,2 x 10⁻¹², tandis qu'un matériau fossile a un ratio nul. En effet, l'isotope ¹⁴C se forme dans l'atmosphère et est ensuite intégré par photosynthèse, selon une échelle de temps de quelques dizaines d'années au plus. La demi-vie du ¹⁴C est de 5730 ans. Donc les matériaux issus de la photosynthèse, à savoir les végétaux de manière générale, ont nécessairement une teneur minimale en isotope ¹⁴C.

La biomasse est donc tout matériau d'origine biologique et ce terme exclut les matériaux enfouis dans les formations géologiques et/ou fossilisées. Des exemples de biomasse sont les plantes, les arbres, les algues, les organismes marins, les micro-organismes, les animaux, etc (tout ou partie de ces organismes). Les matériaux renouvelables sont composés de biomasse et peuvent être reconstitués continuellement.

La teneur en biomatériau ou teneur en carbone renouvelable est déterminée en application de la norme NF EN 16640 qui a pour objet *« Produits biosourcés -Teneur en carbone biosourcé - Détermination de la teneur en carbone biosourcé par la méthode au radiocarbone ».*

Cette Norme Européenne décrit une méthode permettant de déterminer la teneur en carbone biosourcé dans des produits à partir du mesurage de la teneur en ¹⁴C. Elle spécifie également deux méthodes d'essai à utiliser pour déterminer la teneur en 14C, à partir de laquelle la teneur en carbone biosourcé est calculée :
- Méthode A : compteur à scintillation liquide (CSL) ;
- Méthode B : spectrométrie de masse par accélérateur (SMA).

Une troisième méthode, Méthode C : ionisation bêta (IB) peut être également utilisée pour déterminer la teneur en ¹⁴C.

La teneur en carbone biosourcé est exprimée en fraction de masse d'échantillon ou en fraction de la teneur en carbone total.

Les méthodes d'essai et d'analyse spécifiées dans la Norme Européenne EN 16640:2017 sont compatibles avec les méthodes décrites dans l'ASTM D 6866-12.

Dans la molécule de difluorure de vinylidène selon l'invention, le ratio isotopique de ¹⁴C/¹²C est d'au moins 1,2x10⁻¹⁴.

L'invention a pour objet, selon un deuxième aspect, un procédé de préparation du composé selon l'invention, utilisant des produits d'origine naturelle comme produits de départ pour la fabrication de difluorure de vinylidène.

Selon un mode de réalisation, le produit de départ est l'éthylène obtenu à partir de l'éthanol produit directement à partir de la biomasse. On fermente une biomasse à glucides (telle que céréales, betterave) facilement hydrolysable à l'aide d'une levure (par exemple *Saccharomyces Cerevisiae*) ou d'une bactérie (par exemple *Zymomonas* ou *Clostridium*)*.* Une autre voie permet de produire de l'éthanol à partir d'une biomasse ligno-cellulosique (bois, canne à sucre, paille). De tels procédés sont connus de l'homme du métier. Ils comprennent par exemple la fermentation des matières végétales en présence d'une ou plusieurs levures, suivie d'une distillation permettant de récupérer l'éthanol sous forme de solution aqueuse plus concentrée qui est ensuite traitée en vue d'augmenter encore sa concentration molaire en éthanol. L'éthanol obtenu par fermentation est ensuite déshydraté dans un premier réacteur en un mélange d'éthylène et d'eau. On préfère que l'alcool soit injecté en tête du premier réacteur. Cette étape de déshydratation est généralement conduite en présence d'un catalyseur, qui peut notamment être à base de γ-alumine. A titre d'exemple, il a été démontré qu'un ratio du débit volumique d'éthanol liquide au volume de catalyseur de 1 h⁻¹ et une température moyenne du lit catalytique de 400 °C conduisaient à une conversion quasi-totale de l'éthanol avec une sélectivité en éthylène de l'ordre de 98 % mol. La déshydratation peut aussi être effectuée en présence de vapeur d'eau, qui sert alors aussi de fluide caloporteur compensant la consommation de chaleur de la réaction de déshydratation qui est endothermique.

Le procédé selon l'invention comprend une étape de fourniture d'éthylène biosourcé ayant une teneur en carbone renouvelable d'au moins 1 % atomique et la transformation par synthèse en plusieurs étapes en difluorure de vinylidène biosourcé. La première étape de synthèse est la fabrication de chlorure de vinyle monomère (CVM) à partir de cet éthylène biosourcé. La synthèse de CVM se fait par oxychloration de l'éthylène en présence d'oxygène et d'acide chlorhydrique, ou par chloration directe de l'éthylène en présence de dichlore (Cl₂) suivi d'une distillation et d'un craquage à 500 °C puis d'une nouvelle distillation afin de séparer le dichloroéthane du chlorure de vinyle. On trouve une description détaillée du procédé de synthèse du CV dans les Techniques de l'ingénieur - références J6250V1 (10 Juin 1993), et J6020-J1 143V1 (10 septembre 1984). On peut aussi produire le CVM par hydrochloration de l'acétylène, comme indiqué ci-après.

Plusieurs voies de synthèse sont ensuite possibles.

Selon un mode de réalisation, le chlorure de vinyle biosourcé est transformé en VDF biosourcé via les intermédiaires de synthèse suivants : 1,1,2-trichloroéthane, 1,1,1-trichloroéthane et 1-chloro-1,1-difluoroéthane.

Les étapes de réaction sont indiquées ci-dessous :
- fabrication du T112 biosourcé (1,1,2-trichloroéthane ou CH₂Cl-CHCl₂) par chloration froide (avec Cl₂) à partir du CVM biosourcé :

   CH₂=CHCl + Cl₂ → CH₂Cl-CHCl₂
- fabrication de T111 biosourcé (1,1,1-trichloroéthane ou CCl₃-CH₃) à partir du T112 biosourcé par réaction avec HCl gaz, par exemple en passant par le CH₂=CCl₂ (CV2) selon différentes variantes par déshydrochloration à la soude ou à la chaux, puis hydrochloration du CV2 en T111 :

   CH₂Cl-CHCl₂ → CCl₃-CH₃
- fabrication de 142 b (1,1,1-chlorodifluoroéthane ou CH₃-CF₂Cl) biosourcé à partir du T111 biosourcé et réaction avec HF :

   CCl₃-CH₃ + 2 HF → CH₃-CF₂Cl + 2HCl
- fabrication de difluorure de vinylidène biosourcé (VDF ou CF₂=CH₂) à partir du 142 b biosourcé par déshydrochloration thermique :

   CH₃-CF₂Cl → CF₂=CH₂ + HCl

Selon un mode de réalisation, le chlorure de vinyle biosourcé est transformé en VDF biosourcé via les intermédiaires de synthèse suivants : 1,1,2-trichloroéthane, 1,1-dichloroéthène, et 1-chloro-1,1-difluoroéthane.

Les étapes de réaction sont indiquées ci-dessous :
- fabrication du T112 biosourcé (1,1,2-trichloroéthane ou CH₂Cl-CHCl₂) par chloration froide (avec Cl₂) à partir du CVM biosourcé :

   CH₂=CHCl + Cl₂ → CH₂Cl-CHCl₂
- fabrication du CV2 biosourcé (1,1-dichloroéthène ou CH₂=CCl₂) à partir du T112 biosourcé par déshydrochloration par NaOH (ou à la chaux), avec production de saumure :

   CH₂Cl-CH₂Cl₂ + NaOH → CH₂=CCl₂ + NaCl + H₂O
- fabrication de 142 b (1,1,1-chlorodifluoroéthane ou CH₃-CF₂Cl) biosourcé à partir du CV2 biosourcé par fluoration :

   CH₂=CCl₂ + 2HF → CH₃-CF₂Cl + HCl
- fabrication de difluorure de vinylidène biosourcé (VDF ou CF₂=CH₂) à partir du 142 b biosourcé par déshydrochloration thermique :

   CH₃-CF₂Cl → CF₂=CH₂ + HCl

Selon un mode de réalisation, le chlorure de vinyle biosourcé est transformé en VDF biosourcé via les intermédiaires de synthèse suivants : 1,1-dichloréthane, 1,1,1-trichloroéthane et 1-chloro-1,1-difluoroéthane.

Les étapes de réaction sont indiquées ci-dessous :
- fabrication du D11 biosourcé (1,1-dichloroéthane ou CHCl₂-CH₃) à partir du CVM biosourcé par hydrochloration :

   CH₂=CHCl + HCl → CHCl₂-CH₃
- fabrication de T111 biosourcé (1,1,1-trichloroéthane ou CCl₃-CH₃) à partir du D11 biosourcé par chloration :

   CHCl₂-CH₃ + Cl₂ → CCl₃-CH₃ + HCl
- fabrication de 142 b (1,1,1-chlorodifluoroéthane ou CH₃-CF₂Cl) biosourcé à partir du T111 biosourcé et réaction avec HF :

   CCl₃-CH₃ + 2HF → CH₃-CF₂Cl + 2HCl
- fabrication de difluorure de vinylidène biosourcé (VDF ou CF₂=CH₂) à partir du 142 b biosourcé par déshydrochloration thermique :

   CH₃-CF₂Cl → CF₂=CH₂ + HCl

Chacune de ces trois voies peux aussi être utilisée en partant de la réaction d'acétylène biosourcé avec de l'acide chlorhydrique pour fabriquer du CVM biosourcé. L'acétylène biosourcé est fabriqué à partir de biogaz contenant du méthane et/ou à partir de carbure de calcium ayant lui-même été préparé à partir d'une source de carbone renouvelable (charbon de bois, lignine ou autres). Un tel procédé partant d'acétylène est décrit dans les Techniques de l'Ingénieur, référence J 6250 et aussi dans le document FR 2939132.

Un autre objet de l'invention est un homopolymère de difluorure de vinylidène (PVDF) biosourcé comprenant des unités de fluorure de vinylidène biosourcé. L'homopolymérisation du VDF est généralement réalisée par des procédés tels que la suspension ou l'émulsion. Néanmoins, la synthèse de PVDF peut être effectuée en solution ou bien en masse.

L'homopolymère de VDF selon l'invention comporte des monomères VDF provenant de ressources renouvelables, et éventuellement des monomères provenant de ressources fossiles. Lorsque le PVDF est synthétisé à partir d'un mélange de monomères (biosourcés et d'origine fossile), au moins 20 % en poids du mélange est représenté par les unités de monomère biosourcé.

Avantageusement, le PVDF hompolymère ne comporte que des unités de VDF d'origine renouvelable déterminée selon la norme EN 16640:2017.

L'invention concerne également des copolymères fluorés biosourcés comprenant des unités de difluorure de vinylidène biosourcé et un ou plusieurs types d'unités de co-monomères compatibles avec le difluorure de vinylidène. Ces comonomères peuvent être halogénés (fluorés, chlorés ou bromés) ou non-halogénés.

Des exemples de comonomères fluorés appropriés sont : le fluorure de vinyle (VF), le tétrafluoroéthylène (TFE), l'hexafluoropropylène (HFP), les trifluoropropènes et notamment le 3,3,3-trifluoropropène, les tétrafluoropropènes et notamment le 2,3,3,3-tétrafluoropropène ou le 1,3,3,3-tétrafluoropropène, l'hexafluoroisobutylène, le perfluorobutyléthylène, les pentafluoropropènes et notamment le 1,1,3,3,3-pentafluoropropène ou le 1,2,3,3,3-pentafluoropropène, les perfluoroalkylvinyléthers et notamment ceux de formule générale Rf-O-CF-CF₂, Rf étant un groupement alkyle, de préférence en C1 à C4 (des exemples préférés étant le perfluoropropylvinyléther ou PPVE et le perfluorométhylvinyléther ou PMVE). Le monomère fluoré peut comporter un atome de chlore ou de brome. Il peut en particulier être choisi parmi le bromotrifluoroéthylène, le chlorofluoroethylène, le chlorotrifluoroéthylène et le chlorotrifluoropropène. Le chlorofluoroéthylène peut désigner soit le 1-chloro-1-fluoroéthylène, soit le 1-chloro-2-fluoroéthylène. L'isomère 1-chloro-1-fluoroéthylène est préféré. Le chlorotrifluoropropène est de préférence le 1-chloro-3,3,3-trifluoropropène ou le 2-chloro-3,3,3-trifluoropropène.

Le copolymère fluoré biosourcé peut aussi comprendre des monomères non halogénés tels que l'éthylène, notamment l'éthylène biosourcé, et/ou des comonomères acryliques ou méthacryliques.

La présente invention a également pour objet l'utilisation du PVDF biosourcé et/ou des copolymèrés fluorés biosourcés conformes à ceux définis précédemment dans diverses applications où l'aspect de ces polymères est essentiel et/ou leurs propriétés dépendent de leur degré de pureté ; il s'agit des applications dans le génie chimique, l'automobile, la filtration des fluides, notamment de l'eau potable, l'off-shore, le médical, le transport d'eau potable, le marché du semi-conducteur, la câblerie, les batteries Li-ion, le photovoltaïque, les articles de sport et les textiles pour le sport.

Des applications préférées sont dans l'électronique, notamment pour la fabrication des appareils électroniques grand public : équipement audio et vidéo pour usage domestique et commercial, jeux électroniques et équipement de divertissement, équipement de bowling et de billard, équipement de communication par câble et par satellite, composants électroniques utilisés dans les équipements audio et vidéo, équipement de télévision en circuit fermé et instruments de musique.

Ces applications nécessitent la mise en œuvre à l'état fondu du PVDF, comme par exemple l'extrusion, l'injection ou l'extrusion de film par les techniques de soufflage de gaine ou à plat. Lors de ces mises en œuvre les températures habituellement utilisées sont comprises entre 190 et 260°C, avec des temps de résidence dans les équipements pouvant varier de quelques dizaines de secondes (typiquement 30 s) jusqu'à plusieurs minutes, une durée de 10, voire 15 minutes n'étant pas inhabituelle. Ceci peut entraîner une dégradation thermique du polymère fluoré avec, comme conséquence visible, son jaunissement, traduit par un indice de jaune élevé, par exemple supérieur à 15 en YI plaque 230°C, 10 minutes. Ce phénomène est lié à la présence d'impuretés dans le polymère fluoré, notamment des résidus de mercure et d'arsenic, qui contaminent les matières premières, notamment l'éthylène d'origine fossile, utilisées pour la synthèse du VDF.

Le difluorure de vinylidène biosourcé étant exempt de ces impuretés, tout polymère le contenant aura avantageusement un degré de pureté plus élevé, ce qui permet lors de la transformation à haute température d'éviter un jaunissement du PVDF.

Avantageusement, le PVDF biosourcé et/ou copolymère fluorés biosourcés de l'invention sont utilisés seuls ou en mélange avec d'autres polymères, lesdits polymères fluorés biosourcés représentant en masse de 5 à 100%, de préférence de 5 à 70%, de préférence de 5 à 30%.

## Revendications

1. Procédé de préparation de difluorure de vinylidène ayant une teneur en carbone renouvelable est d'au moins 1 % atomique comme déterminé par la teneur en ¹⁴C selon la norme NF EN 16640, et comprenant une étape de fourniture d'acétylène ayant une teneur en carbone renouvelable d'au moins 1%, et la transformation par synthèse en plusieurs étapes en difluorure de vinylidène biosourcé, dans lequel la première étape de synthèse consiste en la fabrication de chlorure de vinyle monomère (CVM) biosourcé à partir de l'acétylène biosourcé.

2. Procédé selon la revendication 1, dans lequel la teneur en bio-carbone est supérieure à 5%, de préférence supérieure à 10%, de préférence supérieure à 25%, de préférence supérieure ou égale à 33%, de préférence supérieure à 50%, de préférence supérieure ou égale à 66%, de préférence supérieure à 75%, de préférence supérieure à 90%, de préférence supérieure à 95%, de préférence supérieure à 98%, de préférence supérieure à 99%, avantageusement égale à 100%.

3. Procédé selon l'une des revendications 1 ou 2, comprenant la suite d'étapes suivantes :
- fabrication du T112 biosourcé (1,1,2-trichloroéthane) par chloration froide avec Cl₂ à partir du CVM biosourcé :
CH₂=CHCl + Cl₂ → CH₂Cl-CHCl₂
- fabrication de T111 biosourcé (1,1,1-trichloroéthane) à partir du T112 biosourcé :
CH₂Cl-CHCl₂ → CCl₃-CH₃
- fabrication de 142 b (1,1,1-chlorodifluoroéthane) biosourcé à partir du T111 biosourcé et réaction avec HF :
CCl₃-CH₃ + 2 HF → CH₃-CF₂Cl + 2HCl
- fabrication de difluorure de vinylidène biosourcé (VDF ou CF₂=CH₂) à partir du 142 b biosourcé par déshydrochloration thermique :
CH₃-CF₂Cl → CF₂=CH₂ + HCl

4. Procédé selon l'une des revendications 1 ou 2, comprenant la suite d'étapes suivantes :
- fabrication du T112 biosourcé (1,1,2-trichloroéthane) par chloration froide avec Cl₂ à partir du CVM biosourcé :
CH₂=CHCl + Cl₂ → CH₂Cl-CHCl₂
- fabrication du CV2 biosourcé (1,1-dichloroéthène ou CH₂=CCl₂) à partir du T112 biosourcé par déshydrochloration par NaOH, avec production de saumure :
CH₂Cl-CH₂Cl₂ + NaOH → CH₂=CCl₂ + NaCl + H₂O
- fabrication de 142 b (1,1,1-chlorodifluoroéthane) biosourcé à partir du CV2 biosourcé par fluoration :
CH₂=CCl₂ + 2HF → CH₃-CF₂Cl + HCl
- fabrication de difluorure de vinylidène biosourcé (VDF ou CF₂=CH₂) à partir du 142 b biosourcé par déshydrochloration thermique :
CH₃-CF₂Cl → CF₂=CH₂ + HCl

5. Procédé selon l'une des revendications 1 ou 2, comprenant la suite d'étapes suivantes :
- Fabrication du D11 biosourcé (1,1-dichloroéthane) à partir du CVM biosourcé par hydrochloration :
CH₂=CHCl + HCl → CHCl₂-CH₃
- fabrication de T111 biosourcé (1,1,1-trichloroéthane) à partir du D11 biosourcé par chloration :
CHCl₂-CH₃ + Cl₂ → CCl₃-CH₃ + HCl
- fabrication de 142 b (1,1,1-chlorodifluoroéthane) biosourcé à partir du T111 biosourcé et réaction avec HF :
CCl₃-CH₃ + 2HF → CH₃-CF₂Cl + 2HCl
- fabrication de difluorure de vinylidène biosourcé (VDF ou CF₂=CH₂) à partir du 142 b biosourcé par déshydrochloration thermique :
CH₃-CF₂Cl → CF₂=CH₂ + HCl
